# EUROPEAN PATENT APPLICATION

(11) **EP 1 076 093 A1**
(43) Date of publication of application: **14.02.2001**
(21) Application number: 00906693.7
(22) Date of filing: 03.03.2000
(51) Int. Cl.: C12N 15/54, C12N 9/10, C12N 15/63, C12N 1/15, C12N 1/19, C12N 1/21, C12N 5/00

(54) **SAKURANETIN SYNTHASE GENE**

(30) Priority: 04.03.1999 JP 5774899
(71) Applicant: Japan Science and Technology Corporation, Kawaguchi-shi, Saitama 332-0012 (JP)
(72) Inventor: KODAMA, Osamu, Inashiki-gun, Ibaraki 300-1151 (JP)
(74) Representative: Woods, Geoffrey Corlett
(86) International application number: JP0001306
(87) International publication number: WO0052174

(57) **Abstract**

DNAs as specified below. (a) A DNA containing the base sequence represented by SEQ ID NO:6. (b) A DNA containing a base sequence derived from the sequence represented by SEQ ID NO:6 by deletion, substitution or addition of one or more bases and being capable of expressing a protein having a naringenin 7-O-methyltrasnferase activity when transferred into rice cells. A rice NOMT genome DNA and a DNA substantially having the function of the former DNA (namely, a function of expressing a protein having an NOMT activity when transferred into a rice cell). Since a member of plants have naringenin serving as the substrate of NOMT, transfer of such a DNA makes it possible to derive sakuranetin from naringenin in plants thus impart an excellent antibacterial characteristic to the plants.

## Description

### FIELD OF THE INVENTION

The present invention relates to genomic DNA of rice-derived sakuranetin synthase (naringenin-7-O-methyltransferase, hereinafter, referred to as "NOMT") and to a promoter region of the genomic DNA.

### BACKGROUND OF THE INVENTION

Plants have inducible resistance to invasion of pathogen, such as production of phytoalexin.

Phytoalexin is a low-molecular-weight antipathogenic substance which is produced and accumulated not only by pathogen invasion but also due to chemical and physical stresses.

In the case of rice (*Oryza sativa*), for example, sakuranetin (5,4*'*- dihydroxy-7-methoxyflavanone), a major phytoalexin of rice, is produced and accumulated in response to pathogen infection and stresses such as UV irradiation, copper chloride (CuCl₂) treatment and jasmonic acid treatment.

Sakuranetin is one of flavonoids which are generally considered to act as phytoalexin to protect plants from stresses such as UV irradiation. O-methyltransferase is an enzyme involved in biosynthesis of such flavonoids, which has been isolated and purified from various plant tissues and cultured cells (Pakusch AE et al., *Arch. Biochem*. *Biophys*. 271:488-494 (1989); Wanek W et al., Planta. 197:427-434 (1995)).

O-methyltransferase is also involved in biosynthesis of sakuranetin. Sakuranetin is synthesized by methylating hydroxy group at position 7 of naringenin (a fravanone, 5,7,4*'*-trihydroxyfravanone) by NOMT.

Since naringenin exists in various plants, sakuranetin may be induced in a plant by introducing DNA that is capable of expressing NOMT into the plant, so that the plant could acquire a remarkable antibacterial property.

However, DNA that is capable of expressing NOMT upon introduction into a plant has not yet been isolated nor identified.

Thus, there has been a demand for isolating and identifying DNA capable of expressing NOMT upon introduction into a plant.

### SUMMARY OF THE INVENTION

The present invention aims at providing DNA capable of expressing NOMT upon introduction into a plant.

We have done intensive studies to solve the above-described problem, and succeeded in identifying amino acid sequences of a N-terminal region(SEQ ID NO:4) and a C-terminal region(SEQ ID NO:5) of NOMT isolated and purified from rice that had been placed under a stress of, for example, UV irradiation, jasmonic acid treatment or copper chloride treatment. PCR was performed using the total genomic DNA of rice as a template, and DNA primers F1, F2, R1 and R2 designed on the above-mentioned partial amino acid sequences of NOMT. The fragment successfully amplified with primers F2 and R2 by PCR was used as a probe for screening a BAC (bacterial artificial chromosome) library of rice genome. As a result, genomic DNA of rice NOMT (SEQ ID NO:6) was successfully isolated and identified, thereby achieving the present invention.

Specifically, the present invention provides the following (i) to (xii).
(i) (a) DNA comprising the nucleotide sequence of SEQ ID NO:6; or (b) DNA having one or more nucleotides deleted, substituted or added in the nucleotide sequence of SEQ ID NO:6, the DNA being capable of expressing a protein having a NOMT activity upon introduction into rice or other plant cells.
(ii) A recombinant vector comprising the DNA of (i).
(iii) A host cell transformed with the recombinant vector of (ii).
(iv) A plant transformant obtained by differentiation of a plant cell, introduced with the recombinant vector of (ii).
(v) (a) DNA comprising the nucleotide sequence of SEQ ID NO:12; or (b) DNA having one or more nucleotides deleted, substituted or added in the nucleotide sequence of SEQ ID NO:12, the DNA being capable of expressing a protein having a NOMT activity upon introduction into a rice cell or other plant cells.
(vi) A recombinant vector comprising the DNA of (v).
(vii) A host cell transformed with the recombiant vector of (vi).
(viii) A plant transformant obtained by differentiation of a plant cell introduced with the recombinant vector of (vi).
(ix) (a) DNA comprising the nucleotide sequence of SEQ ID NO:2; or (b) DNA having one or more nucleotides deleted. substituted or added in the nucleotide sequence of SEQ ID NO:2, the DNA having a promoter activity.
(x) A recombinant vector comprising the DNA of (ix).
(xi) A host cell transformed with the recombinant vector of (x).
(xii) (a) A protein comprising the amino acid sequence of SEQ ID NO:3; or (b) a protein having one or more amino acids deleted, substituted or added in the nucleotide sequence of SEQ ID NO:3, and having a NOMT activity.

This specification includes all or part of the contents as disclosed in the specification of Japanese Patent Application No. 11-57748, which is a priority document of the present application.

### BRIEF DESCRIPTION OF THE DRAWINGS

Figure 1 shows positions of introns and exons of genomic DNA (SEQ ID NO:1) of rice NOMT obtained in Example 3;
Figure 2 shows the expected binding positions of primers F2 and R2 in the genomic DNA (SEQ ID NO:1) of rice NOMT obtained in Example 3.

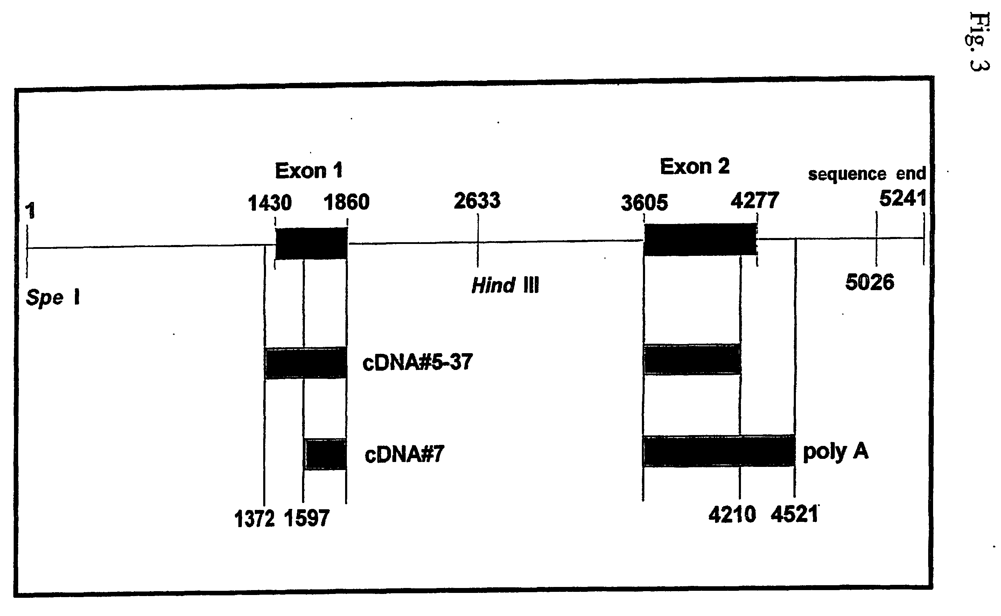

Figure 3 shows positions of exons of genomic DNA (SEQ ID NO:6) of rice NOMT obtained in Example 4;

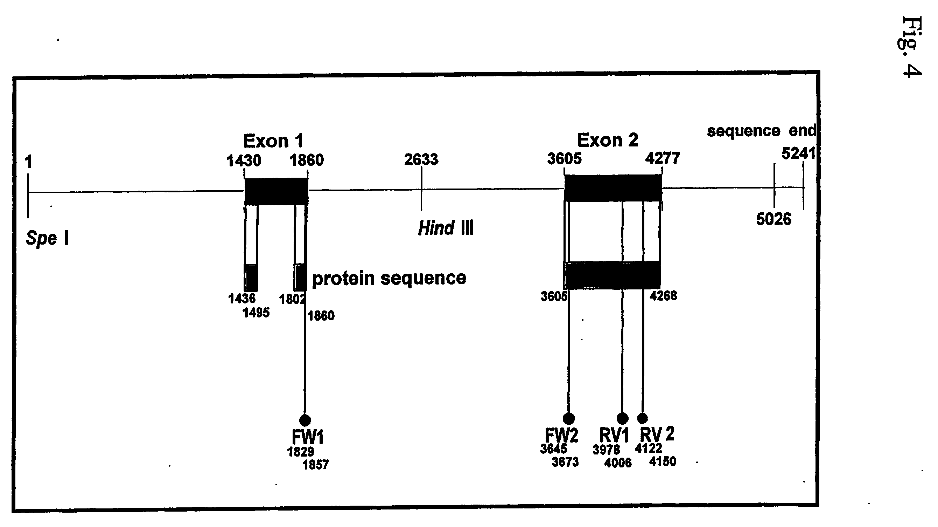

Figure 4 shows protein coding regions in genomic DNA (SEQ ID NO:6) of rice NOMT obtained in Example 4;
Figure 5 shows comparison between the amino acid sequence (SEQ ID NO:3) deduced from the genomic sequence and the partial amino acid sequences (SEQ ID NOS:4 and 5) obtained from the primary structure of the purified protein; and
Figure 6 shows the sequencing process of the genomic DNA of rice NOMT (SEQ ID NO:6).

### DETAILED DESCRIPTION OF THE INVENTION

Hereinafter, the present invention will be described in more detail.

The first aspect of the present invention is:
(a) DNA comprising the nucleotide sequence of SEQ ID NO:6; or
(b) DNA having one or more nucleotides deleted substituted or added in the nucleotide sequence of SEQ ID NO:6, the DNA being capable of expressing a protein having a NOMT activity upon introduction into a rice cell or other plant cells.

DNA comprising the nucleotide sequence represented by SEQ ID NO:6 may be obtained, for example, through Steps (1) to (3) below:

### (1) Construction of DNA library of rice genome

A DNA library of rice genome may be prepared according to a common method. As the DNA library, a BAC (bacterial artificial chromosome) library of rice genome will be most convenient to use. An example of the BAC library of rice genome includes the BAC library stocked by the Laboratory of Disease Physiology, the National Institute of Agrobiological Resources, Japanese Ministry of Agriculture and Forestry, which includes at least 7 equivalents of rice (*Shimokita*) genomes. Since the BAC library of rice (*Shimokita*) genome is sorted into about 20,000 clones, a clone of interest may readily and efficiently be taken out therefrom.

### (2) Selection of clones containing genomic DNA of rice NOMT

A clone containing genomic DNA of rice NOMT may be selected, for example, by screening the BAC library of rice genome according to a common method of membrane hybridization by using a probe made of a nucleotide sequence that can specifically hybridize to the genomic DNA of rice NOMT. The nucleotide sequence of the probe used in the above-mentioned method may be determined based on the amino acid sequence (SEQ ID NO:3) of rice NOMT and such probe may be chemically synthesized according to a common method, or may be PCR-amplified with the primers of F1, F2, R1, and R2, and rice genome as the template.

### (3) Isolation/identification of genomic DNA of rice NOMT

Southern blotting analysis is performed on the fragments of the selected BAC clone after digestion with some appropriate restriction enzymes, by using the above-described probe to identify a genomic DNA fragment of rice NOMT in the clone. Then, the identified fragment is cloned again in a suitable vector. The nucleotide sequence may be determined by Maxam-Gilbert method, Sanger method, or a modification thereof for carrying out such methods automatically. Herein, binary vector pBIGRZ, which can accommodate a large insert, may be used for stably maintaining a large fragment having a promoter region and for directly transforming a plant.

DNA having one or more nucleotides deleted, substituted or added in the nucleotide sequence of SEQ ID NO:6, and being capable of expressing a protein having an NOMT activity upon introduction into rice or other plant cells may be obtained by a technique commonly employed at the time of filing this application,, for example, by site-directed mutagenesis (*Nucleic Acids Res*. 10, 6487-6500, 1982). Specifically, the genomic DNA of rice NOMT is denatured into a single strand form. The single strand is in turn annealed with an oligonucleotide which is designed to introduce a mutation into the single strand, thereby making double strands with polymerase or the like. Then, a strand introduced with the mutation is selected to obtain the DNA of interest.

The number and the positions of the nucleotides that are deleted, substituted or added in the nucleotide sequence of SEQ ID NO:6 are not particularly limited as long as the nucleotide is capable of expressing a protein with an NOMT activity upon introduction into the rice or other plant cells.

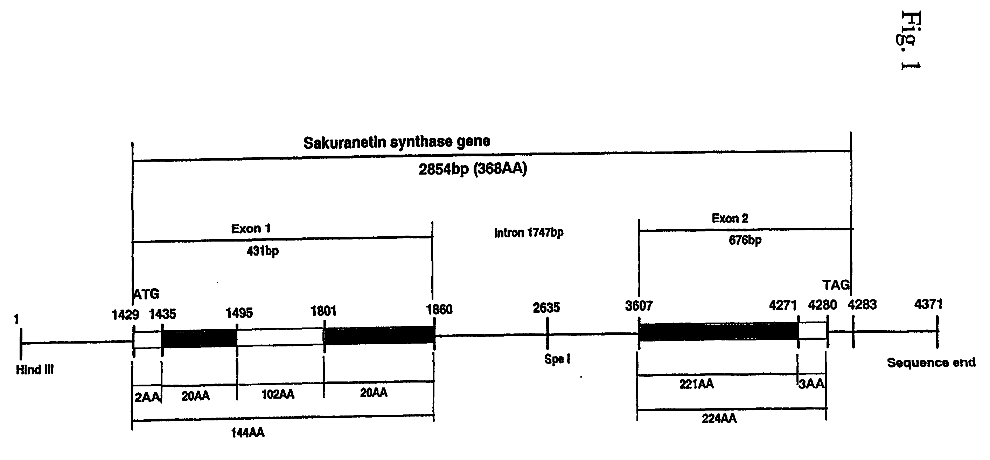

The nucleotide sequence of SEQ ID NO:1 or 6 includes a promoter region and NOMT-coding regions. The promoter region corresponds to a nucleotide region 1-1428 (SEQ ID NO:2) of the nucleotide sequence of SEQ ID NO:1 obtained in Example 3 below, and NOMT-coding regions correspond to nucleotide regions 1429-1859 and 3607-4279 of the nucleotide sequence of SEQ ID NO:1 (Fig.1). The amino acid sequence of NOMT coded by the NOMT-coding regions is represented by SEQ ID NO:3.

Accordingly, "deletion, substitution or addition" in the nucleotide sequence of SEQ ID NO:6 may refer to "deletion, substitution or addition" in the promoter region and/or the NOMT-coding regions.

The number and the positions of the nucleotides deleted, substituted or added in the promoter region are not particularly limited as long as the promoter activity of the promoter region is retained.

The number and the positions of the nucleotides deleted, substituted or added in the NOMT-coding regions are not particularly limited as long as the coding regions code a protein having an NOMT activity. Examples of the protein having the NOMT activity include a protein having the amino acid sequence of SEQ ID NO:3, as well as proteins having one or more amino acids deleted, substituted or added in the amino acid sequence of SEQ ID NO:3, and still having the NOMT activity.

Since DNA of the first aspect of the invention includes a promoter region and regions coding for a protein having an NOMT activity, a protein having an NOMT activity may efficiently be expressed in a suitable host cell by introducing a suitable vector including the DNA of the first aspect of the invention into the host cell.

The vector for incorporating the DNA of the first aspect of the invention is not particularly limited. For example, pUC18, pUC19, pBluescript, pBR322, pBI121, pBIGRZ, TAC or the like may be used.

In order to facilitate the detection of the transformant introduced with the vector, suitable marker or reporter genes may be inserted into the vector beforehand. Examples of such marker gene include genes which confers resistance against antibiotics such as tetracycline, ampicillin, kanamycin, neomycin, hygromycin and spectinomycin. Examples of the reporter gene include genes coding for β-glucuronidase (GUS), chloramphenicol acetyltransferase (CAT), luciferase (LUX), Green fluorescence protein (GFP) and the like.

The host cell for introducing the vector is not particularly limited as long as the host cell is compatible with and is capable of being transformed with the vector including the DNA of the first aspect of the invention. Various cells such as generally-used natural cells as well as artificially-established recombinant cells may be used. For example, plant cells (e.g., rice, cucumber, tomato, barley, potato, corn), animal cells (e.g., mouse, rat, chicken), insect cells (e.g., silk worm), mould (e.g., *Aspergillus*), bacteria (e.g., *E.coli*, *Bacillus subtilis*) and yeast may be used. The vector may be introduced into the host cell by a known method such as a protoplast method, a lithium method, electroporation, a calcium chloride method, or a modification thereof.

After the vector including the DNA of the first aspect of the invention is introduced into a plant cell, the plant cell is differentiated to obtain recombinant plants having the DNA of the first aspect of the invention in each cell.

The gene is introduced into a plant cell by, for example, a method using *Agrobacterium*, electroporation, polyethylene glycol method, microinjection, microparticle bombardment method, but the method is not limited thereto and any method may be employed as long as the method is capable of introducing a gene into a target plant cell.

The species of the host plant is not particularly limited as long as it is compatible with and is capable of being tranformed with the vector including the DNA of the first aspect of the invention. For example, dicotyledons such as cucumber, tomato, Chinese cabbage, potato, cabbage, soybean and rapeseed, and monocotyledons such as rice, barley, corn and wheat may be used.

The plant cells which are introduced with the vector including the DNA of the first aspect of the invention may be differentiated according to a common method. For example, when the gene is introduced into the plant cell by a leaf disc technique, a leaf disc collected from a sterile leaf of a sterilely-cultured plant is immersed in a culture solution containing *Agrobacterium tumefaciens* EHA101, then cultured in a foliage differentiating medium to form and proliferate calli. The foliage differentiating medium may be obtained by supplementing a plant hormone (e.g., 2,4-D, NAA, kinetin) to a known medium such as an MS medium. A callus is selected by using the foliage differentiating medium for selection. The selection medium may be obtained by supplementing, for example, kanamycin, cefotaxime or the like to the foliage differentiating medium. The plant cell may be further differentiated by culturing in a root differentiating medium made by supplementing kanamycin, cefotaxime or the like to a known medium such as an MS medium. Thereafter, the rooted seedling is transferred to soil for growing into a plant.

The protein having the NOMT activity which can be expressed by introducing the DNA of the first aspect of the invention into a host plant may be used to methylate hydroxy group at position 7 of naringenin for synthesizing sakuranetin that may function as phytoalexin. In other words, introduction of the DNA of the first aspect of the invention into a plant induces sakuranetin from naringenin in plant, by which the plant is considered to obtain a remarkable antifungal property.

The second aspect of the present invention is:
(a) DNA comprising the nucleotide sequence of SEQ ID NO:12; or
(b) DNA having one or more nucleotides deleted, substituted or added in the nucleotide sequence of SEQ ID NO:12, the DNA being capable of expressing a protein having a NOMT activity upon introduction into rice or other plant cells, yeast, fungi, or even in bacteria.

The nucleotide sequence shown in SEQ ID NO:12 is cDNA of rice NOMT. The DNA comprising the nucleotide sequence shown in SEQ ID NO:12 may be obtained by, for example, preparing at first the mRNA fraction from the rice green leaves stressed with CuCl₂ , or UV irradiation, using for example the oligo-dT beads of latex or magnetic beads as described in Plant Molecular Biology Manual D5 1-13 (Edited by ST Gelvin, RA Schilperoort: 1994), and then the cDNAs are synthesized with the reverse transcriptase using the above mRNA as the template and oligo dT as the primer with the known method as described for example in Current Protocols in Molecular Biology; Section 5.5 (John & Wiley). An oligo-DNA adaptor may be ligated to the 5*'* end of the mRNA to make possible to set a primer for making a double strand DNA for cloning into an appropriate vector to clone the double stranded cDNA. Then the cDNA clones are transformed to the competent E.Coli by electro-poration, and a cDNA library is constructed. The colony-blotted membranes of the CDNA library may be hybridized to detect the NOMT clone, with the probes PCR-amplified with the F2 and R2 primer set and the rice genome DNA as the template.

In this case, two long fragments of cDNA clones were obtained. They were overlapping in the long central part and complementing the other clone's lacking parts of the 5*'*- and 3'- terminal regions, respectively.

DNA having one or more nucleotides deleted, substituted or added in the nucleotide sequence of SEQ ID NO:12, and being capable of expressing a protein having an NOMT activity upon introduction into rice or other plant cells may be obtained by a technique commonly employed at the time of filing this application, for example, by site-directed mutagenesis (*Nucleic Acids Res*. 10, 6487-6500, 1982). Specifically, the genomic DNA of rice NOMT is denatured into a single strand form. The single strand is in turn annealed with an oligonucleotide which is designed to introduce a mutation into the single strand, thereby making double strands with polymerase or the like. Then, a strand introduced with the mutation is selected to obtain the DNA of interest.

The number and the positions of the nucleotides that are deleted, substituted or added in the nucleotide sequence of SEQ ID NO:12 are not particularly limited as long as the nucleotide is capable of expressing a protein with an NOMT activity upon introduction into the rice or other plant cells.

The number and the positions of the nucleotides deleted, substituted or added in the NOMT-coding regions are not particularly limited as long as the coding regions code a protein having an NOMT activity. Examples of the protein having the NOMT activity include a protein having the amino acid sequence of SEQ ID NO:3, as well as proteins having one or more amino acids deleted, substituted or added in the amino acid sequence of SEQ ID NO:3, and still having the NOMT activity.

Since DNA of the second aspect of the invention includes regions coding for a protein having an NOMT activity, a protein having an NOMT activity may efficiently be expressed in a suitable host cell by introducing a suitable vector including the DNA of the second aspect of the invention into the host cell. In this case, a regulator sequence such as a promoter or an enhancer which aids efficient expression may appropriately used. Such regulator sequence may readily be selected and used by those skilled in the art.

The vector for incorporating the DNA of the second aspect of the invention is not particularly limited. For example, pUC18, pUC19, pBluescript, pBR322, pBI121, pBIGRZ, TAC, pET156 or the like may be used.

In order to facilitate the detection of the transformant introduced with the vector, suitable marker or reporter genes may be inserted into the vector beforehand. Examples of such marker gene include genes which confers resistance against antibiotics such as tetracycline, ampicillin, kanamycin, neomycin, hygromycin and spectinomycin. Examples of the reporter gene include genes coding for β-glucuronidase (GUS), chloramphenicol acetyltransferase (CAT), luciferase (LUX), Green fluorescence protein (GFP) and the like.

The host cell for introducing the vector is not particularly limited as long as the host cell is compatible with and is capable of being transformed with the vector including the DNA of the second aspect of the invention. Various cells such as generally-used natural cells as well as artificially-established recombinant cells may be used. For example, plant cells (e.g., rice, cucumber, tomato, barley, potato, corn), animal cells (e.g., mouse, rat, chicken), insect cells (e.g., silk worm), mould (e.g., *Aspergillus*), bacteria (e.g., *E.coli*, *Bacillus subtilis*) and yeast may be used. The vector may be introduced into the host cell by a known method such as a protoplast method, a lithium method, electroporation, a calcium chloride method, or a modification thereof.

After the vector including the DNA of the second aspect of the invention is introduced into a plant cell, the plant cell is differentiated to obtain recombinant plants having the DNA of the second aspect of the invention in each cell.

The gene is introduced into a plant cell by, for example, a method using *Agrobacterium*, electroporation, polyethylene glycol method, microinjection, microparticle bombardment method, but the method is not limited thereto and any method may be employed as long as the method is capable of introducing a gene into a target plant cell.

The species of the host plant is not particularly limited as long as it is compatible with and is capable of being tranformed with the vector including the DNA of the second aspect of the invention. For example, dicotyledons such as cucumber, tomato, Chinese cabbage, potato, cabbage, soybean and rapeseed, and monocotyledons such as rice, barley, corn and wheat may be used.

The plant cells which are introduced with the vector including the DNA of the second aspect of the invention may be differentiated according to a common method. For example, when the gene is to be introduced into the plant cell by a leaf disc technique, a leaf disc collected from a sterile leaf of a sterilely-cultured plant is immersed in a culture solution containing *Agrobacterium tumefaciens* EHA101, then cultured in a foliage differentiating medium to form and proliferate calli. The foliage differentiating medium may be obtained by supplementing a plant hormone (e.g., 2,4-D, NAA, kinetin) to a known medium such as art MS medium. A callus is selected by using the foliage differentiating medium for selection. The selection medium may be obtained by supplementing, for example, kanamycin, cefotaxime or the like to the foliage differentiating medium. The plant cell may be further differentiated by culturing in a root differentiating medium made by supplementing kanamycin, cefotaxime or the like to a known medium such as an MS medium. Thereafter, the rooted seedling is transferred to soil for growing into a plant.

The protein having the NOMT activity which can be expressed by introducing the DNA of the second aspect of the invention into a host plant may be used to methylate hydroxy group at position 7 of naringenin for synthesizing sakuranetin that may function as phytoalexin. In other words, introduction of the DNA of the second aspect of the invention into a plant induces sakuranetin from naringenin in plant, by which the plant is considered to obtain a remarkable antifungal property.

The third aspect of the present invention is:
(a) DNA comprising the nucleotide sequence of SEQ ID NO:2; or
(b) DNA having one or more nucleotides deleted, substituted or added in the nucleotide sequence of SEQ ID NO:2, the DNA having a promoter activity.

DNA comprising the nucleotide sequence of SEQ ID NO:2 may be obtained by determining and isolating a promoter region from the nucleotide sequence (SEQ ID NO:1) of genomic DNA of rice NOMT.

The promoter region of the genomic DNA of rice NOMT may be determined according to a common method, for example, by comparing the nucleotide sequence of the gene obtained from a cDNA library with a nucleotide sequence obtained from the library of the gemonic DNA.

The promoter region may be isolated by using a suitable restriction enzyme. For example, genomic DNA of rice NOMT may be digested partially by restriction enzymes such as *Hind*III*, EcoR*I, and *Sau*IIIAI for isolating a DNA fragment containing the promoter region.

The DNA comprising the nucleotide sequence of SEQ ID NO:2 may be obtained by PCR using rice genomic DNA as a template, and a nucleotide sequence complementary to 5*'*-end of the nucleotide sequence of SEQ ID NO:2 and a nucleotide sequence complementary to 3*'*-end of the nucleotide sequence of SEQ ID NO:2 as promoters.

DNA comprising a nucleotide sequence having one or more nucleotides deleted, substituted or added in the nucleotide sequence of SEQ ID NO:2, and having a promoter activity may be obtained by a technique commonly-employed at the time of filing the present application such as site-directed mutagenesis (*Nucleic Acids Res*. 10, 6487-6500, 1982).

The number and the positions of the nucleotides deleted, substituted or added in the nucleotide sequence of SEQ ID NO: 2 are not particularly limited as long as the promoter activity of the DNA is retained.

Since the DNA of the third aspect of the invention has a promoter activity, a gene of interest can be expressed by introducing, a vector containing the DNA of the third aspect of the invention and the gene of interest linked downstream therefrom, into a host cell.

Herein, the gene of interest refers to a gene coding for a gene product to be expressed (for example, a protein, rRNA or antisense RNA).

The gene of interest which may be linked downstream from the DNA of the invention is not particularly limited and may be any gene, such as chitinase gene, β-1,3 glucanase gene and PAL (phenylalanine ammonialyase) gene.

The vector for incorporating the DNA of the third aspect of the invention is not particularly limited. For example, pUC18, pUC19, pBluescript, pBR322, pBI121, pBIGRZ and TAC may be used.

In order to facilitate the detection of the transformant introduced with the vector, a suitable marker or reporter gene may be inserted downstream or upstream of the DNA of the third aspect of the invention. Examples of the marker gene include a gene which confers resistance against antibiotics such as tetracycline, ampicillin, kanamycin, neomycin, hygromycin and spectinomycin. Examples of the reporter gene include genes coding for β-glucuronidase (GUS), chloramphenicol acetyltransferase (CAT), luciferase (LUX), and the like.

The host cell for introducing the vector is not particularly limited as long as the host cell is compatible with and is capable of being transformed with the vector including the DNA of the third aspect of the invention. various cells such as generally-used natural cells or artificially-established recombinant cells may be used. For example, plant cells (e.g., rice, cucumber, tomato, potato, tobacco), animal cells (e.g., mouse, rat, chicken), insect cells (e.g., silk worm), bacteria (e.g., *E.coli*, *Bacillus subtilis*) and yeast may be used. The vector may be introduced into the host cell by a known method such as a protoplast method, a lithium method, electroporation, a calcium chloride method, or a modification thereof.

The fourth aspect of the present invention is:
(a) a protein comprising the amino acid sequence of SEQ ID N0:3; or
(b) a protein having one or more amino acids deleted, substituted or added in the nucleotide sequence of SEQ ID NO:3, and having a NOMT activity.

The amino acid sequence shown in SEQ ID NO:3 corresponds to the amino acid sequence of rice NOMT protein. The protein having the amino acid sequence shown in SEQ ID NO:3 may be obtained, for example, by overexpressing the cDNA (SEQ ID: No 12) in E.Coli by transforming with the vector comprising the cDNA and an appropriate promoter for expressing the cDNA for example pET15b (Novagen, Madison, WI). The proteins expressed in E. Coli will have the enzyme activity of NOMT as described by XZ He et al. (Plant Molecular Biology 36:43-54, 1998)..

A protein having one or more amino acids deleted, substituted or added in the amino acid sequence of SEQ ID NO:3, and having an NOMT activity may be obtained by a technique commonly employed at the time of filing this application, for example, by modifying the cDNA structure with the site-directed mutagenesis as described in the case of gene modification.

The number and the positions of the amino acids that are deleted, substituted or added in the amino acid sequence of SEQ ID NO:3 are not particularly limited as long as a protein with an NOMT activity is expressed.

### EXAMPLES

### Example 1: Construction of BAC (bacterial artificial chromosome) library of rice genomes

A genomic DNA library derived from a *japonica* rice variety, *Shimokita*, was constructed with a BAC (bacterial artificial chromosome) vector according to the method of *Molecular General Genetics* (1997) Vol.254, pp.611-620.

The constructed BAC library of the rice genomes had an average insert size of about 155 kbp and corresponded about 7 equivalents of genomes. A membrane of one microplate size included 3072 clones (corresponding to about a single genome equivalent of rice). Since the clones are sorted and stocked in this BAC library of rice genome, only a series of membranes are required to be subjected to colony hybridization using a suitable probe to screen a positive clones of interest.

### Example 2: Determination of amino acid sequence of rice NOMT

Rice (*Hitomebore*) was cultivated according to the method of Kodama et al. (Phytochemistry, 31: 3807-3809 (1992)), and then subjected to UV irradiation. The UV-irradiated rice (1 g) was crushed in a mortar in the presence of 4 ml 0.2 M Trig-HCl buffer (pH 7.8) (containing 14 mM mercaptoethanol, 5 mM EDTA, 10%(w/v) glycerol and 10% (w/w) polyvinylpyrrolidone) and 0.05 g sea sand.

The solution containing the crushed rice was subjected to centrifugation at 18,500 x g for 5 minutes. The supernatant was filtrated through a 50 µm nylon membrane, and the filtrate was loaded to adenosine-agarose column (0.7 x 15 cm) which had been equilibrated with Buffer B (0.02 M Tris-HCl (pH 7.8) containing 10% glycerol, 1 mM EDTA-2Na and 14 mM 2-mercaptoethanol).

The adenosine-agarose column equilibrated with Buffer B was prepared as follows. Five ml of 5*'*-AMP-agarose (Sigma) was washed with distilled water and incubated with 800 units of bovine small intestine alkaline phosphatase dissolved in 1 ml of a bovine small intestine alkaline phosphatase buffer (500 mM Tris-HCl (pH 9.0)) (total amount of 10 ml) to dephosphorylate the gel. Incubation was carried out by placing the gel in a vial, followed by continuous rotation at 37°C for 24 hours. The obtained gel was packed into a column, washed with distilled water, then with 10 ml Buffer B containing 2M MaCl, and finally equilibrated with 100 ml of Buffer B, thereby preparing an adenosine-agarose column equilibrated with Buffer B.

The column was washed with 50 ml of Buffer B at a flow rate of 18 ml/h, and then washed with 50 ml of Buffer B containing 0.2 M KCl. NOMT was selectively eluted with 25 ml Buffer B containing 4 mM S-adenosyl-L-methionine (SAM) and 0.2M KCl. The eluted fractions were collected for 1.75 ml each.

The NOMT activity was measured for each fraction as follows.

Forty µl of each fraction, 375 µM naringenin, 0.1 M glycine-sodium hydroxide buffer (pH 9.5) containing 5 mM DTT and 1 mM EDTA were mixed with 92.5 Bq/µl S-[¹⁴C] adenosylmethionine (volume 160 µl) and reacted at 27°C for 20 minutes. After the reaction was completed, 25 µl of 6N hydrochloric acid, and subsequently 1 ml of a toluene scintillator solution containing 0.4% PPO were added to the reaction mixture and well agitated. Thereafter, radioactivity of the generated sakuranetin was measured with a liquid scintillation counter to determine the NOMT activity.

Fractions having the NOMT activity were loaded into Superdex 75 gel filtration Chromato Column (3 x 30 cm) (Pharmacia Biotech) which had been equilibrated with Buffer B to elute NOMT at a flow rate of 18ml/h using the same buffer as eluent. 3.5 ml fractions were collected and the NOMT activity in each fraction was measured as described above, thereby obtaining purified NOMT.

The obtained purified NOMT was concentrated using Centricon-30 (Amicon, Beverly, USA), desalted (2 x 500 µl milliQ purified water), and dried with a spin-dry-concentrator. Dried NOMT (about 10 µg) was dissolved in 200 µl of 70% formic acid containing 1% cyanogen bromide, incubated in a dark place for 20 hours, and dried as described above. The dried product was subjected to TRICINE SDS-PAGE (16.5% T, 3% C gel) (Schagger H et al., *Anal*. *Biochem*. 166:368-379 (1987)), and blotted onto a PVDF membrane (Fluorotrans, Pall, Tokyo, Japan). The stained peptide was cut out from the membrane, mounted on a direct sequencing cartridge to determine the amino acid sequence thereof with AB494 Protein Sequencer.

Consequently, both the N-terminal amino acid sequence (SEQ ID NO:4) and the C-terminal amino acid sequence (SEQ ID NO:5) of NOMT were determined.

### Example 3: Selection of clones containing genomic DNA of rice NOMT

DNA primers were designed based on the partial amino acid sequences of NOMT (SEQ ID NOS: 4 and 5) determined in Example 2. Specifically, two parts, with less genetic code redundancy, were selected from each of the N- and C- terminal amino acid sequences of the purified NOMT. Based on the selected parts, sense primers F1 and F2, and antisense primers R1 and R2 were designed. The nucleotide sequences of primers F1, F2, R1 and R2 (the positions of these primers are indicated in the figure) were as follows:
Primer F1: atgaa(c/t)ca(a/g)ga(c/t)aa(a/g)gtictiatgga(a/g)ag
Primer F2: tt(g/c)aa(c/t)aa(a/g)gcita(c/t)ggiatgacigcitt
Primer R1: tcict(a/g)ca(a/g)tc(a/g)tgiagiat(a/g)ca(c/t)ttcat
Primer R2: agcatiatcat(a/g)tciac(a/g)tg(a/g)aaiac(a/g)cc

In the above-mentioned nucleotide sequences, "i" refers to inosine; while "(g/c)", "(c/t)" and "(a/g)" refer to either guanine or cytosine, either cytosine or thymine, and either adenine or guanine, respectively. For the oligonucleotide synthesis, these degenerating nucleotides were mixed for their positions.

PCR was conducted using total DNA of rice genome as a template and the DNA primers designed as described above.

The composition of the PCR reaction solution was as follows:

| | |
|---|---|
| Takara Ex Taq (5U/µl) | 0.125 µl (0.625U) |
| 10 x buffer | 2.5 µl |
| 2.5 mM dNTP | 2 µl |
| Primer (20 µM) | 2.5 µl |
| Template | 125 ng |
| H₂O | |
| Total | 25µl |

PCR was conducted through: one cycle of heat denaturation (94°C, 3 min.); 29 cycles of heat denaturation (94°C, 1 min.), annealing (65°C, 2 min.) and elongation (72°C, 2 min.); and one cycle of elongation (72°C, 5 min.), and stored at 4°C.

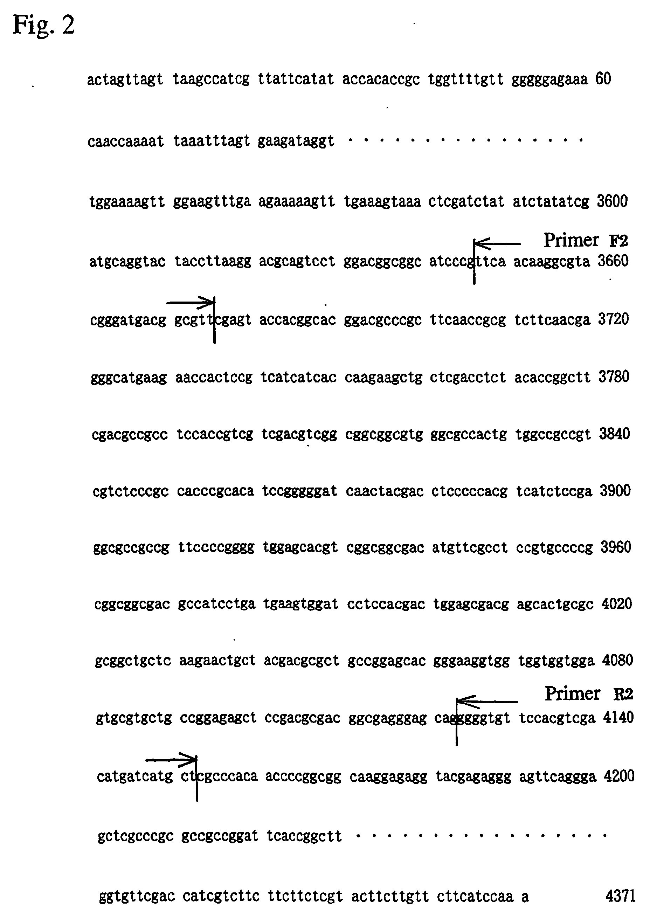

A single band was obtained which had a length that was expected to be the product amplified by PCR with primers F2 and R2. Figure 2 shows the binding positions of primers F2 and R2 in the rice NOMT genomic DNA (SEQ ID NO:1).

The fragment amplified by the above PCR was sequenced to confirm that it was consistent with the amino acid sequence of NOMT. Then, the amplified fragment was labeled with ECL (Amersham) so as to be used as an ECL probe for colony hybridization of the BAC library of rice genome constructed in Example 1. As a result, 6 positive clones were obtained. After Southern blotting analysis with HindIII, 3 clones (25-4c, 7-4D, 58-1A) were found to be authentic.

The clones were cultured, and the obtained BAC plasmids were digested with restriction enzyme *Hind*III, followed by southern hybridization. As a result, a positive band of about 15 kbp with an intensive signal was detected in four clones. The positive bands were excised and introduced into binary vector pBIGRZ which can accommodate a large insert. Since vector pBIGRZ has low copy number, it is capable of stably maintaining an insert of 10 kbp or more in *E.coli* and also in *Agrobacterium*. The inserted portion may directly be introduced into various plants including rice by *Agrobacterium* method. Direct sequencing can be carried out starting from any sites in the insert by using suitable primers.

The obtained clones were sequenced starting from both ends of the vector's cloning site and using the primers synthesized from the sequences of PCR-derived bands. Then, based on the thus-obtained data, new primers were synthesized to further elongate the sequence so as to fill the gaps. A DNA fragment having a nucleotide sequence corresponding to the amino acid sequence of NOMT was obtained from the *Spe*I fragment of the BAC clone (25-4c) about 8 kbp, and inserted in pBIGRZ vector. The sequence of the fragment was determined according to a primer extension method. As a result, a sequence from 5*'*end to Nucleotide 4371, was determined mainly in one direction which contained a full-length NOMT coding region (including an open reading frame and an intron) and a promoter region of about 1.4 kbp. The resulting nucleotide sequence of the genomic DNA of rice NOMT is represented by SEQ ID NO:1. Nucleotide regions 1429-1859 and 3607-4282 were expected to be exons and a nucleotide region 1860-3606 an intron (Figure 1). The amino acid sequence of rice NOMT deduced from the nucleotide sequence of the genomic DNA of rice NOMT is shown in SEQ ID NO:3.

The amino acid sequence of NOMT (SEQ ID NO:3) deduced from the expected nucleotide sequence of the genomic DNA of rice NOMT (SEQ ID NO:1) obtained above was compared with the partial amino acid sequences (SEQ ID NOS: 4 and 5) determined in Example 2 using isolated and purified NOMT derived from UV-exposed rice. As a result, they were mostly consistent with each other but with partial difference. This difference was considered to result from an experimental error in protein sequencing owing to difficult characterstics of the amino acids such as tryptophan(W),cystein(C),methionine(M), the close peaks of the derivatives, and the minute amount of the NOMT isolated and purified in Example 2. Figure 1 shows the relationship between the amino acid sequence of the NOMT isolated and purified in Example 2 and the nucleotide sequence of the genomic DNA of rice NOMT, indicating that the isolated DNA is undoubtedly genomic DNA of rice NOMT. In Figure 1, the black bars represent the amino acid sequences of the NOMT isolated and purified in Example 2.

### Example 4: Determination of genomic DNA sequence of rice NOMT

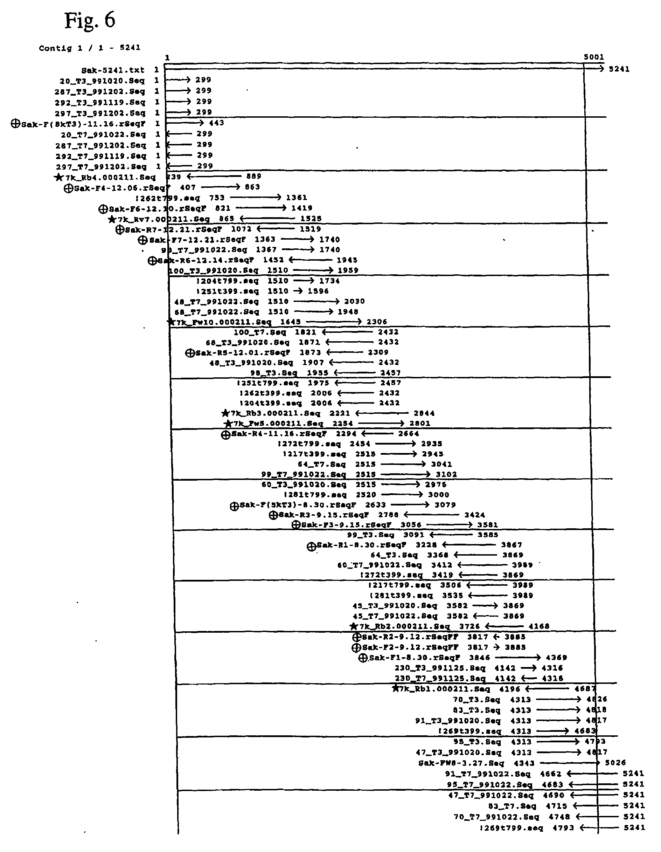

The above insert was partially digested with *Sau*IIIaI which recognizes four bases so as to obtain the reverse direction of the sequences which was not obtained in the above described one series of primer extension method. The fragment of 0.5-2 kB was cloned into plasmid pBSK. Significant lengths of regions were read by random sequencing using T3 and T7 primers on the vector's flanking ends of the insert (Figure 6). Since a number of gaps remained, new primers were designed for further reading the sequence. As a result, residues read in both directions were 5026, and a sum of residues read in both directions and/or in one direction were 5241, containing the 3*'* end sequence (SEQ ID NO:6). Although it differed at 12 sites from the sequence (SEQ ID NO:1) obtained in Example 3 above, no difference was found between the amino acid sequences of the proteins deduced from both sequences.

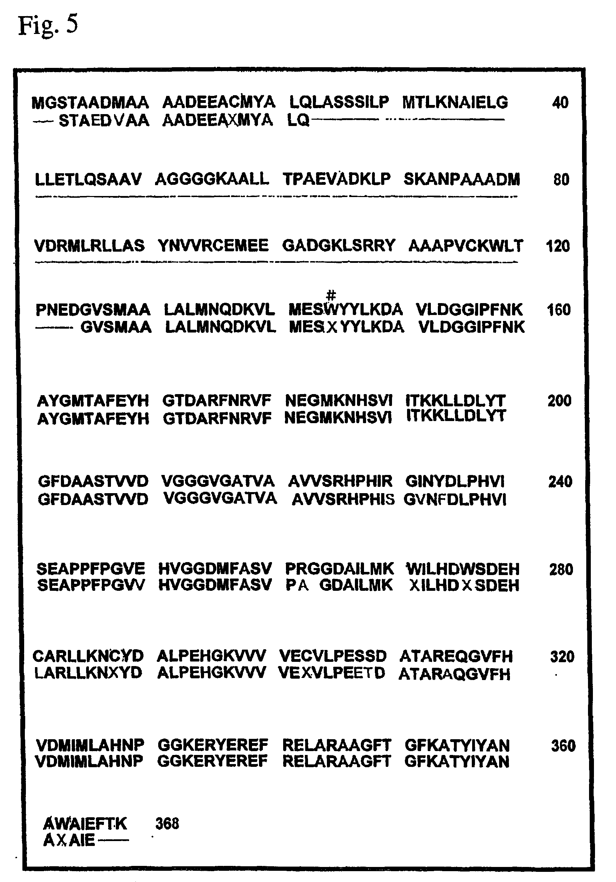

The amino acid sequences (i.e., N-terminal sequence (SEQ ID NO:4) and C-terminal sequence (SEQ ID NO:5)) determined by analyzing the primary structure of the enzyme protein and the sequence deduced from the gene (SEQ ID NO:3) have some differences shown in Figure 5. However, most of these differences result from the fact that it is hard to detect the peaks of W (tryptophan), C (cysteine), M (methionine) and the like. Other than that, 9 residues are different, but I/V, A/E and Y/F also tend to be mistaken. Therefore, only 6 amino acids among the 261 aminoacids determined, are mismatching to the result of DNA sequencing of NOMT. As the material was rather small for protein sequecing, these remaining mismatches may be due to the difficulty in the protein sequencing. Accordingly, the obtained gene is considered to be a sakuranetin synthase gene.

### Example 5: Determination of cDNA sequence of rice NOMT genome

Nucleotide regions 1430-1860 and 3605-4277 in the resulting nucleotide sequence (SEQ ID NO:6) of the genomic DNA of rice NOMT were found to be exons. The nucleotide sequence of cDNA (SEQ ID NO:12) was determined as the following.

The RNA were extracted from the rice (Nipponbare) green leaves with detergents and proteins were removed with phenol extraction, and then precipitated by adding 1 vol of 5 N LiCl and rinsed with 70% EtOH. The redissolved RNA was fractionated with the oligo-dT beads of latex (Roche) as described in the manual. The cDNAS were synthesized with the reverse transcriptase using the above mRNA as the template and oligo dT as the primer. The single strand (as) CDNA was ligated to a single strand DNA adaptor with the T4RNA ligase and PCR amplified with the oligo dT and the adaptor as the primers. Then the cDNAs were ligated to lambda ZAP as the blunt end and transformed to the competent E.Coli, and a cDNA library was constructed. The colony-blotted membranes of the CDNA library may be hybridized to detect the NOMT clone, with the probes PCR-amplified with the F2 and R2 primer set and the rice genome DNA as the template. After a series of purification of the positive plaque, the purified clone was sequenced for its insert by extending sequences from the flanking vector primers.

In this case, two long sequences of cDNA clones cDNA #5-37 and cDNA #7 were obtained. They were overlapping in the long central part (1597-4210; in the SEQ ID 6) and complementing the other clone's lacking parts of the 5*'*- and 3'- terminal regions, respectively.

The present invention provides genomic DNA of rice NOMT and DNA substantially having the function thereof (i.e., the function capable of expressing a protein having an NOMT activity upon introduction into a rice cell). Since naringenin exists in various plants, sakuranetin may be induced from naringenin in plants by introducing the DNA of the invention into the plants. As a result, the plants could acquire a remarkable antifungal property.

The present invention also provides DNA having a promoter activity. Since the DNA of the present invention have a promoter activity, it may be used as a promoter for introducing a foreign gene into a host cell.

All publications and patent applications cited herein are incorporated herein by reference in their entirety.

SEQUENCE LISTING

<110> Japan Science and Technology Corporation

<120> Sakuranetin Synthetase Gene

<130> N.80844 GCW

<160> 12

<210> 1
<211> 4371
<212> DNA
<213> Oryza sativa

<400> 1
actagttagt taagccatcg ttattcatat accacaccgc tggttttgtt gggggagaaa 60
caaccaaaat taaatttagt gaagataggt aggaatatga cagcacataa cgttcacttt 120
gaactcaagc atggacataa ttagttggag aagaaaatga tggtgcccac ttacctagct 180
agctatacag gatatttcaa caaattaaag ggttagcaat tttctgtcaa gtacactttt 240
gttccaaaag ggacagtaat cgtcagttaa ccagtttaac tctcacccat atatgatcaa 300
tcatgttgaa tattttaggc agacataaaa taggagaaat gtcaatttat ccggcttaat 360
taattgctaa attacaatta agttctatgt ggaccatata tatggtttcg tagaaaacaa 420
ttcatgtcca taatataatg aagaatatta gtatcagatt ttaatatcta tcaagttacc 480
catcacagca gtacagtaac tcctaattaa tgttcttgct tcatcacact catatatata 540
cttcctccgt ttcacaacgt aagttattct agcattttcc atatttatat taatgctaat 600
gaatctagac atatatatct atttaaattt attagcatca atatgaatgt ggaaaatgct 660
agaatgactt acattgtgaa acggatggag taccactcaa attacagggc tccaagctac 720

ttaattagtg agcaactaat tggtctactc gatctctcca tcttttggcc accaactcca 780
cacactccaa aataaagctt tcactttcat cccagcacaa tatgcatgca acggaatagt 840
tgtcgagttt gaccgttaac actaaaaaca aaccccatgc acgacttaac atacctaatc 900
gttgaaaata attagccgta aatattacat atatatatat acatcagatg atgaattgta 960
gtaattaaat taattaaacc tactaaaccc atacaacgtc gtcacgacta ctcctgatgg 1020
tacatctaag ctactccatt tcccatcgga cgaatacaac cacgcaccag acaaagtacg 1080
caaacacaaa attatcaatg ataatcatga tgatggagca tcatatctac tactagcaca 1140
tacactatct ttcttccttc cttctcctaa ttaattcccc taaaaaattg aaggtgaaga 1200
gaagaagaag gaaaagaaag caaaacaaat taattaaaga aaagaaaaga aaagaaaaag 1260
gtgagttggt ggtgtcatat atagtgtgtg gtggttggtg aaggcttgag ctcccatata 1320
aaccccctcc tccctctcca ttgccttcac ctcactcgcc gccggtgatc agctcctcct 1380
ccttcttctc catcgccggc gagagagaga gagagttagc tagctaggat gggttctaca 1440
gccgccgaca tggccgcggc ggccgacgag gaggcgtgca tgtacgcgct gcagctggcg 1500
tcgtcgtcga tcctgccgat gacgctcaag aacgccatcg agctgggcct gctcgagacg 1560
ctgcagtccg ccgccgtcgc cggaggaggg gggaaggcgg cgctgctgac gccggcggag 1620
gtggccgaca agctgccgtc caaggcgaac ccggcggcgg ccgacatggt ggaccgcatg 1680
ctccgcctgc tcgcctccta caacgtcgtc aggtgcgaga tggaggaggg cgccgacggc 1740
aagctctccc gccgatacgc cgccgcgccg gtgtgcaagt ggctgacgcc caacgaggac 1800
ggcgtctcca tggccgccct cgccctcatg aaccaggaca aggtcctcat ggagagctgg 1860
tcagttcatt ttgtctcctc ctctctcgct gacatgtggg cccaccccta cataaacctt 1920
cacaaataaa acccactatg ccaacttcca ttttcaaaag gagttaggtg caacaagcat 1980
atcaaacctt gaatttcatt gtttttttta gtttcctttt agaaccttga gacaacatgt 2040
tttttagatg ataggataaa ggaatatccc cggcctctga atcatatgat gcacgtaatc 2100
gattttctaa agaaactaat tccatgaaga aaaatcgttc ttacacgtat aaatagcacc 2160
ggcaggcacg taatcgattt tcaatacata tgctagaaaa gaaattaatt ctctgacgaa 2220
aatttgttct tacgtacgca tagcaccgac tgatagccgc atacggcata attatgtgca 2280
cttagaaaag ggtgttggtg accctcctct agtagtgggg ggtgatgcta gcaagcacta 2340
aacttggtca ccaaactagc tgaggaatat gaaaagaggg gggaaaaggt tgagctcatc 2400
ttccttctag agaagcaacc acagccgtcg atcgccgaat cgacggcgaa tagagatccc 2460

ttcccaatca tcctaacatg tcatgctcaa aatgcatgat ggagaaatct tggctgatct 2520
gatcagctgg cttggttgtc acaaactgat tctccaccta cctgtagaaa attcggtagt 2580
gttgtgtata tatagccagt ttgtttgatg tggacagagt actagaaatc aaataagctt 2640
gtgtgacttt tcctgcatgc ctttgattat tcttctaaga ttttggggtt cagaaattca 2700
ggtagaaagt agaaaaaaga gtacagtagt tattattcag aacatcctgt gcaaagccca 2760
agcaagctgt acccttttag tggcaagatt gcaatggcat gtaggttggt tagtaggtag 2820
tgcgtctaga caaagacaca tcagtagcaa cagcagcagc tggaggagga acatccatca 2880
atttcctacc aaaacaatcc actcacagcc tcacaggcat tcaatggatg cgtggtgtca 2940
attataggat ttgaaccccg ttgaacagcc gacttctttc tcattttctc atgacctcat 3000
agttatcccg ctgaatcttt atatgcttat atcttacaga atatggattg tactccaaaa 3060
tcgttttcac aactttttac aaatttatcg gaccgcctac aacattatta ctctagaaat 3120
tattggttaa agttgcatta tgaagaccgt tcaactccaa gaattattat ttttgcgcat 3180
aaaaagatat tttattttag gaaaaataag gccccatttt ttaagttgaa attgatggaa 3240
tttttctata tctatttcag gtataaacaa atgaagtgta actataacaa agttgaaaaa 3300
acatattttc gaaaatatat aatttttttt acaaaacata ccattagaac attggtaagc 3360
atgcaaataa aaatccataa tctagaatga gaaaagtaca tggacaataa aaatgaagaa 3420
gttgcatatg cctgaatatg gctgtgttta gttcacgcta aaattaaaag tttggttgaa 3480
attagaatga tgtgatggaa aagttggaag tttgtgtgta tagaaaaagt tttgatgtga 3540
tggaaaagtt ggaagtttga agaaaaagtt tgaaagtaaa ctcgatctat atctatatcg 3600
atgcaggtac taccttaagg acgcagtcct ggacggcggc atcccgttca acaaggcgta 3660
cgggatgacg gcgttcgagt accacggcac ggacgcccgc ttcaaccgcg tcttcaacga 3720
gggcatgaag aaccactccg tcatcatcac caagaagctg ctcgacctct acaccggctt 3780
cgacgccgcc tccaccgtcg tcgacgtcgg cggcggcgtg ggcgccactg tggccgccgt 3840
cgtctcccgc cacccgcaca tccgggggat caactacgac ctcccccacg tcatctccga 3900
ggcgccgccg ttccccgggg tggagcacgt cggcggcgac atgttcgcct ccgtgccccg 3960
cggcggcgac gccatcctga tgaagtggat cctccacgac tggagcgacg agcactgcgc 4020
gcggctgctc aagaactgct acgacgcgct gccggagcac gggaaggtgg tggtggtgga 4080
gtgcgtgctg ccggagagct ccgacgcgac ggcgagggag cagggggtgt tccacgtcga 4140
catgatcatg ctcgcccaca accccggcgg caaggagagg tacgagaggg agttcaggga 4200

gctcgcccgc gccgccggat tcaccggctt caaggccacc tacatctacg ccaacgcctg 4260
ggccatcgag ttcaccaagt aggtgattgg tgatcgatcg ccattgttga gctcgatcaa 4320
ggtgttcgac catcgtcttc ttcttctcgt acttcttgtt cttcatccaa a 4371

<210> 2
<211> 1428
<212> DNA
<213> Oryza sativa

<400> 2
actagttagt taagccatcg ttattcatat accacaccgc tggttttgtt gggggagaaa 60
caaccaaaat taaatttagt gaagataggt aggaatatga cagcacataa cgttcacttt 120
gaactcaagc atggacataa ttagttggag aagaaaatga tggtgcccac ttacctagct 180
agctatacag gatatttcaa caaattaaag ggttagcaat tttctgtcaa gtacactttt 240
gttccaaaag ggacagtaat cgtcagttaa ccagtttaac tctcacccat atatgatcaa 300
tcatgttgaa tattttaggc agacataaaa taggagaaat gtcaatttat ccggcttaat 360
taattgctaa attacaatta agttctatgt ggaccatata tatggtttcg tagaaaacaa 420
ttcatgtcca taatataatg aagaatatta gtatcagatt ttaatatcta tcaagttacc 480
catcacagca gtacagtaac tcctaattaa tgttcttgct tcatcacact catatatata 540
cttcctccgt ttcacaacgt aagttattct agcattttcc atatttatat taatgctaat 600
gaatctagac atatatatct atttaaattt attagcatca atatgaatgt ggaaaatgct 660
agaatgactt acattgtgaa acggatggag taccactcaa attacagggc tccaagctac 720
ttaattagtg agcaactaat tggtctactc gatctctcca tcttttggcc accaactcca 780
cacactccaa aataaagctt tcactttcat cccagcacaa tatgcatgca acggaatagt 840
tgtcgagttt gaccgttaac actaaaaaca aaccccatgc acgacttaac atacctaatc 900
gttgaaaata attagccgta aatattacat atatatatat acatcagatg atgaattgta 960
gtaattaaat taattaaacc tactaaaccc atacaacgtc gtcacgacta ctcctgatgg 1020
tacatctaag ctactccatt tcccatcgga cgaatacaac cacgcaccag acaaagtacg 1080
caaacacaaa attatcaatg ataatcatga tgatggagca tcatatctac tactagcaca 1140

tacactatct ttcttccttc cttctcctaa ttaattcccc taaaaaattg aaggtgaaga 1200
gaagaagaag gaaaagaaag caaaacaaat taattaaaga aaagaaaaga aaagaaaaag 1260
gtgagttggt ggtgtcatat atagtgtgtg gtggttggtg aaggcttgag ctcccatata 1320
aaccccctcc tccctctcca ttgccttcac ctcactcgcc gccggtgatc agctcctcct 1380
ccttcttctc catcgccggc gagagagaga gagagttagc tagctagg 1428

<210> 3
<211> 368
<212> PRT
<213> Oryza sativa

<400> 3
Met Gly Ser Thr Ala Ala Asp Met Ala Ala Ala Ala Asp Glu Glu Ala
1               5                   10                  15

Cys Met Tyr Ala Leu Gln Leu Ala Ser Ser Ser Ile Leu Pro Met Thr
            20                  25                  30

Leu Lys Asn Ala Ile Glu Leu Gly Leu Leu Glu Thr Leu Gln Ser Ala
        35                  40                  45

Ala Val Ala Gly Gly Gly Gly Lys Ala Ala Leu Leu Thr Pro Ala Glu
    50                  55                  60

Val Ala Asp Lys Leu Pro Ser Lys Ala Asn Pro Ala Ala Ala Asp Met
65                  70                  75                  80

Val Asp Arg Met Leu Arg Leu Leu Ala Ser Tyr Asn Val Val Arg Cys
                85                  90                  95

Glu Met Glu Glu Gly Ala Asp Gly Lys Leu Ser Arg Arg Tyr Ala Ala
            100                 105                 110

Ala Pro Val Cys Lys Trp Leu Thr Pro Asn Glu Asp Gly Val Ser Met
        115                 120                 125

Ala Ala Leu Ala Leu Met Asn Gln Asp Lys Val Leu Met Glu Ser Trp
    130                 135                 140

Tyr Tyr Leu Lys Asp Ala Val Leu Asp Gly Gly Ile Pro Phe Asn Lys
145                 150                 155                 160

Ala Tyr Gly Met Thr Ala Phe Glu Tyr His Gly Thr Asp Ala Arg Phe
                165                 170                 175

Asn Arg Val Phe Asn Glu Gly Met Lys Asn His Ser Val Ile Ile Thr
            180                 185                 190

Lys Lys Leu Leu Asp Leu Tyr Thr Gly Phe Asp Ala Ala Ser Thr Val
        195                 200                 205

Val Asp Val Gly Gly Gly Val Gly Ala Thr Val Ala Ala Val Val Ser
    210                 215                 220

Arg His Pro His Ile Arg Gly Ile Asn Tyr Asp Leu Pro His Val Ile
225                 230                 235                 240

Ser Glu Ala Pro Pro Phe Pro Gly Val Glu His Val Gly Gly Asp Met

                245                 250                 255

Phe Ala Ser Val Pro Arg Gly Gly Asp Ala Ile Leu Met Lys Trp Ile
            260                 265                 270

Leu His Asp Trp Ser Asp Glu His Cys Ala Arg Leu Leu Lys Asn Cys
        275                 280                 285

Tyr Asp Ala Leu Pro Glu His Gly Lys Val Val Val Val Glu Cys Val
    290                 295                 300

Leu Pro Glu Ser Ser Asp Ala Thr Ala Arg Glu Gln Gly Val Phe His
305                 310                 315                 320

Val Asp Met Ile Met Leu Ala His Asn Pro Gly Gly Lys Glu Arg Tyr
                325                 330                 335

Glu Arg Glu Phe Arg Glu Leu Ala Arg Ala Ala Gly Phe Thr Gly Phe
            340                 345                 350

Lys Ala Thr Tyr Ile Tyr Ala Asn Ala Trp Ala Ile Glu Phe Thr Lys
        355                 360                 365

<210> 4
<211> 20
<212> PRT
<213> Oryza sativa

<220>

<221> unsure
<222> 15

<400> 4
Ser Thr Ala Glu Asp Val Ala Ala Ala Ala Asp Glu Glu Ala Xaa Met
1               5                   10                  15

Tyr Ala Leu Gln
            20

<210> 5
<211> 240
<212> PRT
<213> Oryza sativa

<220>
<221> unsure
<222> 146

<220>
<221> unsure
<222> 151

<220>
<221> unsure
<222> 163

<220>
<221> unsure

<222> 173

<220>
<221> unsure
<222> 237

<400> 5
Gly Val Ser Met Ala Ala Leu Ala Leu Met Asn Gln Asp Lys Val Leu
1               5                   10                  15

Met Glu Ser Xaa Tyr Tyr Leu Lys Asp Ala Val Leu Asp Gly Gly Ile
            20                  25                  30

Pro Phe Asn Lys Ala Tyr Gly Met Thr Ala Phe Glu Tyr His Gly Thr
        35                  40                  45

Asp Ala Arg Phe Asn Arg Val Phe Asn Glu Gly Met Lys Asn His Ser
    50                  55                  60

Val Ile Ile Thr Lys Lys Leu Leu Asp Leu Tyr Thr Gly Phe Asp Ala
65                  70                  75                  80

Ala Ser Thr Val Val Asp Val Gly Gly Gly Val Gly Ala Thr Val Ala
                85                  90                  95

Ala Val Val Ser Arg His Pro His Ile Ser Gly Val Asn Phe Asp Leu
            100                 105                 110

Pro His Val Ile Ser Glu Ala Pro Pro Phe Pro Gly Val Val His Val

        115                 120                 125

Gly Gly Asp Met Phe Ala Ser Val Pro Ala Gly Asp Ala Ile Leu Met
    130                 135                 140

Lys Xaa Ile Leu His Asp Xaa Ser Asp Glu His Leu Ala Arg Leu Leu
145                 150                 155                 160

Lys Asn Xaa Tyr Asp Ala Leu Pro Glu His Gly Lys Val Val Val Val
                165                 170                 175

Glu Xaa Val Leu Pro Glu Glu Thr Asp Ala Thr Ala Arg Ala Gln Gly
            180                 185                 190

Val Phe His Val Asp Met Ile Met Leu Ala His Asn Pro Gly Gly Lys
        195                 200                 205

Glu Arg Tyr Glu Arg Glu Phe Arg Glu Leu Ala Arg Ala Ala Gly Phe
    210                 215                 220

Thr Gly Phe Lys Ala Thr Tyr Ile Tyr Ala Asn Ala Xaa Ala Ile Glu
225                 230                 235                 240

<210> 6
<211> 5241
<212> DNA
<213> Oryza sativa

<400> 6

actagttagt taagccatcg ttattcatat accacaccgc tggttttgtt ggtgggagaa 60
acaaccaaaa ttaaatttag tgaagatagg taggaatatg acagcacata acgttcactt 120
tgaactcaag catggacata attagttgga gaagaaaatg atggtgccca cttacctagc 180
tagctataca ggatatttca acaaattaaa gggttagcaa ttttctgtca agtacacttt 240
tgttccaaaa gggacagtaa tcgtcagtta accagtttaa ctctcaccca tatatgatca 300
atcatgttga atattttagg cagacataaa ataggagaaa tgtcaattta tccggcttaa 360
ttaattgcta aattactaat taagttctat gtggaccata tatatggatt tcgtagaaaa 420
caattcagtc cataatataa tgaagaatat tagtatcaga ttttaatatc tatcaagtta 480
cccatcacag cagtacagta actcctaatt aatgttcttg cttcatcaca ctcatatata 540
tacttcctcc gtttcacaac gtaagttatt ctagcatttt ccatatttat attaatgcta 600
atgaatctag acatatatat ctatttaaat ttattagcat caatatgaat gtggaaaatg 660
ctagaatgac ttacattgtg aaacggatgg agtaccactc aaattacagg gctccaagct 720
acttaattag tgagcaacta attggtctac tcgatctctc catcttttgg ccaccaactc 780
cacacactcc aaaataagct ttcactttca tcccagcaca atatgcatgc aaggaatagt 840
tgtcgagttt gaccgttaac actaaaaaca aaccccatgc acgacttaac atacctaatc 900
gttgaaaata attagccgta aatattacat atatatatat acatcagatg atgaattgta 960
gtaattaaat taattaaacc tactaaaccc atacaacgtc gtcacgacta ctcctgatgg 1020
tacatctaag ctactccatt tcccatcgga cgaatacaac cacgcaccag acaaagtacg 1080
caaacacaaa attatcaatg ataatcatga tgatggagca tcatatctac tactagcaca 1140
tacactatct ttcttccttc cttctcctaa ttaattcccc taaaaaattg aaggtgaaga 1200
gaagaagaag gaaaagaaag caaaacaaat taattaaaga aaagaaaaga aaagaaaaag 1260
gtgagttggt ggtgtcatat atagtgtgtg gtggttggtg aaggcttgag ctcccatata 1320
aaccccctcc tccctctcca ttgccttcac ctcactcgcc gccggtgatc agcctcctcc 1380
tccttcttct ccatcgccgg cgagagagag agagagttag ctagctagga tgggttctac 1440
agccgccgac atggccgcgg cggccgacga ggaggcgtgc atgtacgcgc tgcagctggc 1500
gtcgtcgtcg atcctgccga tgacgctcaa gaacgccatc gagctgggcc tgctcgagac 1560
gctgcagtcc gccgccgtcg ccggaggagg ggggaaggcg gcgctgctga cgccggcgga 1620
ggtggccgac aagctgccgt ccaaggcgaa cccggcggcg gccgacatgg tggaccgcat 1680
gctccgcctg ctcgcctcct acaacgtcgt caggtgcgag atggaggagg gcgccgacgg 1740

caagctctcc cgccgctacg ccgccgcgcc ggtgtgcaag tggctgacgc ccaacgagga 1800
cggcgtctcc atggccgccc tcgccctcat gaaccaggac aaggtcctca tggagagctg 1860
gtcagttcat tttgtctcct cctctctcgc tgacatgtgg gcccacccct acataaacct 1920
tcacaaataa aacccactat gccaacttcc attttcaaaa ggagttaggt gcaacaagca 1980
tatcaaacct tgaatttcat tgtttttttt agtttccttt tagaaccttg agacaacatg 2040
ttttttagat gataggataa aggaatatcc ccggcctctg aatcatatga tgcacgtaat 2100
cgattttcta aagaaactaa ttccatgaag aaaaatcgtt cttacacgta taaatagcac 2160
cgacaggcac gtaatcgatt ttcaatacat atgctagaaa agaaattaat tctctgacga 2220
aaatttgttc ttacgtacgc atagcaccga ctgatagccg ctacggcata attatgtgca 2280
cttagaaaag ggtgttggtg accctcctct agtagtgggg ggtgatgcta gcaagcacta 2340
aacttggtca ccaaacagct gaggaatatg aaaagagggg ggaaaaggtt gagctcatct 2400
tccttctaga gaagcaacca cagccgtcga tcgccgaatc gacggcgaat agagatccct 2460
tcccaatcat cctaacatgt catgctcaaa atgcatgatg gagaaatctt ggctgatctg 2520
atcagctggc ttggtgtcac aaactgattc tccacctacc tgtagaaaat tcggtagtgt 2580
tgtgtatata tagccagttt gtttgatgtg gacagagtac tagaaatcaa ataagcttgt 2640
gtgacttttc ctgcatgcct ttgattattc ttctaagatt ttggggttca gaaattcagg 2700
tagaaagtag aaaaaagagt acagtagtta ttattcagaa catcctgtgc aaagcccaag 2760
caagctgtac ccttttagtg gcaagattgc aatggcatgt aggttggtta gtaggtagtg 2820
cgtctagaca aagacacatc agtagcaaca gcagcagctg gaggaggaac atccatcaat 2880
ttcctaccaa aacaatccac tcacagcctc acaggcattc aatggatgcg tggtgtcaat 2940
tataggattt gaaccccgtt gaacagccga cttctttctc attttctcat gacctcatag 3000
ttatcccgct gaatctttat atgcttatat cttacagaat atggattgta ctccaaaatc 3060
gttttcacaa ctttttacaa atttatcgga ccgcctacaa cattattact ctagaaatta 3120
ttggttaaag ttgcattatg aagaccgttc aactccaaga attattattt ttgcgcataa 3180
aaagatattt tattttagga aaaataaggc cccatttttt aagttgaaat tgatggaatt 3240
tttctatatc tatttcaggt ataaacaaat gaagtgtaac tataacaaag ttgaaaaaac 3300
atattttcga aaatatataa ttttttttac aaaacatacc attagaacat tggtaagcat 3360
gcaaataaaa atccataatc tagaatgaga aaagtacatg gacaataaaa atgaagaagt 3420
tgcatatgcc tgaatatggc tgtgtttagt tcacgctaaa attaaaagtt tggttgaaat 3480

tagaatgatg tgatggaaaa gttggaagtt tgtgtgtata gaaaaagttt tgatgtgatg 3540
gaaaagttgg aagtttgaag aaaaagtttg aaagtaaact cgatctatat ctatatcgat 3600
gcaggtacta ccttaaggac gcagtcctgg acggcggcat cccgttcaac aaggcgtacg 3660
ggatgacggc gttcgagtac cacggcacgg acgcccgctt caaccgcgtc ttcaacgagg 3720
gcatgaagaa ccactccgtc atcatcacca agaagctgct cgacctctac accggcttcg 3780
acgccgcctc caccgtcgtc gacgtcggcg gcggcgtggg cgccactgtg gccgccgtcg 3840
tctcccgcca cccgcacatc cgggggatca actacgacct cccccacgtc atctccgagg 3900
cgccgccgtt ccccggggtg gagcacgtcg gcggcgacat gttcgcctcc gtgccccgcg 3960
gcggcgacgc catcctgatg aagtggatcc tccacgactg gagcgacgag cactgcgcgc 4020
ggctgctcaa gaactgctac gacgcgctgc cggagcacgg gaaggtggtg gtggtggagt 4080
gcgtgctgcc ggagagctcc gacgcgacgg cgagggagca gggggtgttc cacgtcgaca 4140
tgatcatgct cgcccacaac cccggcggca aggagaggta cgagagggag ttcagggagc 4200
tcgcccgcgc cgccggattc accggcttca aggccaccta catctacgcc aacgcctggg 4260
ccatcgagtt caccaagtag gtgattggtg atcgatcgcc attgttgagc tcgatcaagg 4320
tgttcgacca tcgtcttctt cttctcgtac ttcttgttct tcatccaaaa gcgtgggtaa 4380
tcatgtcgtt tgctgctggc tgatgctgct gctgcttgtg ttgtactttt gatgttcaat 4440
tccggtgatt ctgagttcta atggatgtaa cctgtctgct attatatata tatatataat 4500
cgaatatatg atatatttac ttacgacttg ttaatgattc acaaggttat ataatttctt 4560
ctcaatcaat cacagcctct atctatttaa ttctacctaa tttcttaatg attttgtcca 4620
aaactaaatt ttcttatatt ctgtggcaga gatagtagtg aatatctaga acaacaaatc 4680
gaaatcatag catcacatta tgagtaaaac aacagctcct tatattatac tacatgatat 4740
tttttttacg caaaaggtag agagactcta ccttgttacc attgtagtgc tccctccgtt 4800
tcaaaatgtt tgacaccgtt gactttttag tacgtgtttg accattcgtt ttattcaaaa 4860
aatttaagta attatttatt cttttcatat catttgattc attgttaaat atactttcat 4920
gtacacatat aattttactt atttcataaa tttttttgaa taaggcgaac ggtcaaacat 4980
gtgctaaaaa gtcaactgtg tcaaacattt tgaaacggag ggagtatgta gtagtagtag 5040
tagtaaaaat actacatgat atgatggtat gtttttgata tgttgttcta gagatttaga 5100
gtaatgtgtc tgcgtgttca gagtttcaga gaggaaacgg aatctttgta ttgctgctgc 5160
tacgtgttga atcgtgcaca attctaggat aaatttacta tcgacttgtt aataattcac 5220

aaggttatat agatttcgat c 5241

<210> 7

<211> 5241

<212> DNA

<213> Oryza sativa

<220>

<221> exon

<222> 1430..1860

<220>

<221> intron

<222> 1861..3604

<220>

<221> exon

<222> 3605..4277

<400> 7

actagttagt taagccatcg ttattcatat accacaccgc tggttttgtt ggtgggagaa 60

acaaccaaaa ttaaatttag tgaagatagg taggaatatg acagcacata acgttcactt 120

tgaactcaag catggacata attagttgga gaagaaaatg atggtgccca cttacctagc 180

tagctataca ggatatttca acaaattaaa gggttagcaa ttttctgtca agtacacttt 240

tgttccaaaa gggacagtaa tcgtcagtta accagtttaa ctctcaccca tatatgatca 300

atcatgttga atattttagg cagacataaa ataggagaaa tgtcaattta tccggcttaa 360

ttaattgcta aattactaat taagttctat gtggaccata tatatggatt tcgtagaaaa 420

caattcagtc cataatataa tgaagaatat tagtatcaga ttttaatatc tatcaagtta 480

cccatcacag cagtacagta actcctaatt aatgttcttg cttcatcaca ctcatatata 540

tacttcctcc gtttcacaac gtaagttatt ctagcatttt ccatatttat attaatgcta 600
atgaatctag acatatatat ctatttaaat ttattagcat caatatgaat gtggaaaatg 660
ctagaatgac ttacattgtg aaacggatgg agtaccactc aaattacagg gctccaagct 720
acttaattag tgagcaacta attggtctac tcgatctctc catcttttgg ccaccaactc 780
cacacactcc aaaataagct ttcactttca tcccagcaca atatgcatgc aaggaatagt 840
tgtcgagttt gaccgttaac actaaaaaca aaccccatgc acgacttaac atacctaatc 900
gttgaaaata attagccgta aatattacat atatatatat acatcagatg atgaattgta 960
gtaattaaat taattaaacc tactaaaccc atacaacgtc gtcacgacta ctcctgatgg 1020
tacatctaag ctactccatt tcccatcgga cgaatacaac cacgcaccag acaaagtacg 1080
caaacacaaa attatcaatg ataatcatga tgatggagca tcatatctac tactagcaca 1140
tacactatct ttcttccttc cttctcctaa ttaattcccc taaaaaattg aaggtgaaga 1200
gaagaagaag gaaaagaaag caaaacaaat taattaaaga aaagaaaaga aaagaaaaag 1260
gtgagttggt ggtgtcatat atagtgtgtg gtggttggtg aaggcttgag ctcccatata 1320
aaccccctcc tccctctcca ttgccttcac ctcactcgcc gccggtgatc agcctcctcc 1380
tccttcttct ccatcgccgg cgagagagag agagagttag ctagctagg atg ggt tct 1438
Met Gly Ser
1
aca gcc gcc gac atg gcc gcg gcg gcc gac gag gag gcg tgc atg tac 1486
Thr Ala Ala Asp Met Ala Ala Ala Ala Asp Glu Glu Ala Cys Met Tyr
    5                   10                  15
gcg ctg cag ctg gcg tcg tcg tcg atc ctg ccg atg acg ctc aag aac 1534
Ala Leu Gln Leu Ala Ser Ser Ser Ile Leu Pro Met Thr Leu Lys Asn
20                  25                  30                  35
gcc atc gag ctg ggc ctg ctc gag acg ctg cag tcc gcc gcc gtc gcc 1582
Ala Ile Glu Leu Gly Leu Leu Glu Thr Leu Gln Ser Ala Ala Val Ala
                40                  45                  50
gga gga ggg ggg aag gcg gcg ctg ctg acg ccg gcg gag gtg gcc gac 1630
Gly Gly Gly Gly Lys Ala Ala Leu Leu Thr Pro Ala Glu Val Ala Asp
            55                  60                  65

aag ctg ccg tcc aag gcg aac ccg gcg gcg gcc gac atg gtg gac cgc 1678
Lys Leu Pro Ser Lys Ala Asn Pro Ala Ala Ala Asp Met Val Asp Arg
        70                  75                  80
atg ctc cgc ctg ctc gcc tcc tac aac gtc gtc agg tgc gag atg gag 1726
Met Leu Arg Leu Leu Ala Ser Tyr Asn Val Val Arg Cys Glu Met Glu
    85                  90                  95
gag ggc gcc gac ggc aag ctc tcc cgc cgc tac gcc gcc gcg ccg gtg 1774
Glu Gly Ala Asp Gly Lys Leu Ser Arg Arg Tyr Ala Ala Ala Pro Val
100                 105                 110                 115
tgc aag tgg ctg acg ccc aac gag gac ggc gtc tcc atg gcc gcc ctc 1822
Cys Lys Trp Leu Thr Pro Asn Glu Asp Gly Val Ser Met Ala Ala Leu
                120                 125                 130
gcc ctc atg aac cag gac aag gtc ctc atg gag agc tg gtcagttcat ttt 187
3
Ala Leu Met Asn Gln Asp Lys Val Leu Met Glu Ser Tr
135 140
gtctcctcct ctctcgctga catgtgggcc cacccctaca taaaccttca caaataaaac 1933
ccactatgcc aacttccatt ttcaaaagga gttaggtgca acaagcatat caaaccttga 1993
atttcattgt tttttttagt ttccttttag aaccttgaga caacatgttt tttagatgat 2053
aggataaagg aatatccccg gcctctgaat catatgatgc acgtaatcga ttttctaaag 2113
aaactaattc catgaagaaa aatcgttctt acacgtataa atagcaccga caggcacgta 2173
atcgattttc aatacatatg ctagaaaaga aattaattct ctgacgaaaa tttgttctta 2233
cgtacgcata gcaccgactg atagccgcta cggcataatt atgtgcactt agaaaagggt 2293
gttggtgacc ctcctctagt agtggggggt gatgctagca agcactaaac ttggtcacca 2353
aacagctgag gaatatgaaa agagggggga aaaggttgag ctcatcttcc ttctagagaa 2413
gcaaccacag ccgtcgatcg ccgaatcgac ggcgaataga gatcccttcc caatcatcct 2473
aacatgtcat gctcaaaatg catgatggag aaatcttggc tgatctgatc agctggcttg 2533
gtgtcacaaa ctgattctcc acctacctgt agaaaattcg gtagtgttgt gtatatatag 2593
ccagtttgtt tgatgtggac agagtactag aaatcaaata agcttgtgtg acttttcctg 2653

catgcctttg attattcttc taagattttg gggttcagaa attcaggtag aaagtagaaa 2713

aaagagtaca gtagttatta ttcagaacat cctgtgcaaa gcccaagcaa gctgtaccct 2773

tttagtggca agattgcaat ggcatgtagg ttggttagta ggtagtgcgt ctagacaaag 2833

acacatcagt agcaacagca gcagctggag gaggaacatc catcaatttc ctaccaaaac 2893

aatccactca cagcctcaca ggcattcaat ggatgcgtgg tgtcaattat aggatttgaa 2953

ccccgttgaa cagccgactt ctttctcatt ttctcatgac ctcatagtta tcccgctgaa 3013

tctttatatg cttatatctt acagaatatg gattgtactc caaaatcgtt ttcacaactt 3073.

tttacaaatt tatcggaccg cctacaacat tattactcta gaaattattg gttaaagttg 3133

cattatgaag accgttcaac tccaagaatt attatttttg cgcataaaaa gatattttat 3193

tttaggaaaa ataaggcccc attttttaag ttgaaattga tggaattttt ctatatctat 3253

ttcaggtata aacaaatgaa gtgtaactat aacaaagttg aaaaaacata ttttcgaaaa 3313

tatataattt tttttacaaa acataccatt agaacattgg taagcatgca aataaaaatc 3373

cataatctag aatgagaaaa gtacatggac aataaaaatg aagaagttgc atatgcctga 3433

atatggctgt gtttagttca cgctaaaatt aaaagtttgg ttgaaattag aatgatgtga 3493

tggaaaagtt ggaagtttgt gtgtatagaa aaagttttga tgtgatggaa aagttggaag 3553

tttgaagaaa aagtttgaaa gtaaactcga tctatatcta tatcgatgca g g tac 3608

p Tyr

145

tac ctt aag gac gca gtc ctg gac ggc ggc atc ccg ttc aac aag gcg 3656

Tyr Leu Lys Asp Ala Val Leu Asp Gly Gly Ile Pro Phe Asn Lys Ala

150 155 160

tac ggg atg acg gcg ttc gag tac cac ggc acg gac gcc cgc ttc aac 3704

Tyr Gly Met Thr Ala Phe Glu Tyr His Gly Thr Asp Ala Arg Phe Asn

165 170 175

cgc gtc ttc aac gag ggc atg aag aac cac tcc gtc atc atc acc aag 3752

Arg Val Phe Asn Glu Gly Met Lys Asn His Ser Val Ile Ile Thr Lys

180 185 190

aag ctg ctc gac ctc tac acc ggc ttc gac gcc gcc tcc acc gtc gtc 3800

Lys Leu Leu Asp Leu Tyr Thr Gly Phe Asp Ala Ala Ser Thr Val Val

195 200 205
gac gtc ggc ggc ggc gtg ggc gcc act gtg gcc gcc gtc gtc tcc cgc 3848
Asp Val Gly Gly Gly Val Gly Ala Thr Val Ala Ala Val Val Ser Arg
210 215 220 225
cac ccg cac atc cgg ggg atc aac tac gac ctc ccc cac gtc atc tcc 3896
His Pro His Ile Arg Gly Ile Asn Tyr Asp Leu Pro His Val Ile Ser
230 235 240
gag gcg ccg ccg ttc ccc ggg gtg gag cac gtc ggc ggc gac atg ttc 3944
Glu Ala Pro Pro Phe Pro Gly Val Glu His Val Gly Gly Asp Met Phe
245 250 255
gcc tcc gtg ccc cgc ggc ggc gac gcc atc ctg atg aag tgg atc ctc 3992
Ala Ser Val Pro Arg Gly Gly Asp Ala Ile Leu Met Lys Trp Ile Leu
260 265 270
cac gac tgg agc gac gag cac tgc gcg cgg ctg ctc aag aac tgc tac 4040
His Asp Trp Ser Asp Glu His Cys Ala Arg Leu Leu Lys Asn Cys Tyr
275 280 285
gac gcg ctg ccg gag cac ggg aag gtg gtg gtg gtg gag tgc gtg ctg 4088
Asp Ala Leu Pro Glu His Gly Lys Val Val Val Val Glu Cys Val Leu
290 295 300 305
ccg gag agc tcc gac gcg acg gcg agg gag cag ggg gtg ttc cac gtc 4136
Pro Glu Ser Ser Asp Ala Thr Ala Arg Glu Gln Gly Val Phe His Val
310 315 320
gac atg atc atg ctc gcc cac aac ccc ggc ggc aag gag agg tac gag 4184
Asp Met Ile Met Leu Ala His Asn Pro Gly Gly Lys Glu Arg Tyr Glu
325 330 335
agg gag ttc agg gag ctc gcc cgc gcc gcc gga ttc acc ggc ttc aag 4232
Arg Glu Phe Arg Glu Leu Ala Arg Ala Ala Gly Phe Thr Gly Phe Lys
340 345 350
gcc acc tac atc tac gcc aac gcc tgg gcc atc gag ttc acc aag tag 4280

Ala Thr Tyr Ile Tyr Ala Asn Ala Trp Ala Ile Glu Phe Thr Lys
355                 360                 365
gtgattggtg atcgatcgcc attgttgagc tcgatcaagg tgttcgacca tcgtcttctt 4340
cttctcgtac ttcttgttct tcatccaaaa gcgtgggtaa tcatgtcgtt tgctgctggc 4400
tgatgctgct gctgcttgtg ttgtactttt gatgttcaat tccggtgatt ctgagttcta 4460
atggatgtaa cctgtctgct attatatata tatatataat cgaatatatg atatatttac 4520
ttacgacttg ttaatgattc acaaggttat ataatttctt ctcaatcaat cacagcctct 4580
atctatttaa ttctacctaa tttcttaatg attttgtcca aaactaaatt ttcttatatt 4640
ctgtggcaga gatagtagtg aatatctaga acaacaaatc gaaatcatag catcacatta 4700
tgagtaaaac aacagctcct tatattatac tacatgatat tttttttacg caaaaggtag 4760
agagactcta ccttgttacc attgtagtgc tccctccgtt tcaaaatgtt tgacaccgtt 4820
gactttttag tacgtgtttg accattcgtt ttattcaaaa aatttaagta attatttatt 4880
cttttcatat catttgattc attgttaaat atactttcat gtacacatat aattttactt 4940
atttcataaa tttttttgaa taaggcgaac ggtcaaacat gtgctaaaaa gtcaactgtg 5000
tcaaacattt tgaaacggag ggagtatgta gtagtagtag tagtaaaaat actacatgat 5060
atgatggtat gtttttgata tgttgttcta gagatttaga gtaatgtgtc tgcgtgttca 5120
gagtttcaga gaggaaacgg aatctttgta ttgctgctgc tacgtgttga atcgtgcaca 5180
attctaggat aaatttacta tcgacttgtt aataattcac aaggttatat agatttcgat 5240
c                                                                 5241

<210> 8
<211> 29
<212> DNA
<213> Artificial Sequence

<220>
<223> Synthetic primer for PCR

<400> 8

atgaaycarg ayaargtnct natggarag 29

<210> 9

<211> 29

<212> DNA

<213> Artificial Sequence

<220>

<223> Synthetic primer for PCR

<400> 9

ttsaayaarg cntayggnat gacngcntt 29

<210> 10

<211> 29

<212> DNA

<213> Artificial Sequence

<220>

<223> Synthetic primer for PCR

<400> 10

tcnctrcart crtgnagnat rcayttcat 29

<210> 11

<211> 29

<212> DNA

<213> Artificial Sequence

<220>

<223> Synthetic primer for PCR

<400> 11

agcatnatca trtcnacrtg raanacrcc 29

<210> 12

<211> 1467

<212> DNA

<213> Oryza sativa

<400> 12

aattcgagga tccgggtacc atgggcctcc tcctccttct tctccatcgc cggcgagaga 60

gagagagagt tagctagcta ggatgggttc tacagccgcc gacatggccg cggcggccga 120

cgaggaggcg tgcatgtacg cgctgcagct ggcgtcgtcg tcgatcctgc cgatgacgct 180

caagaacgcc atcgagctgg gcctgctcga gacgctgcag tccgccgccg tcgccggagg 240

aggggggaag gcggcgctgc tgacgccggc ggaggtggcc gacaagctgc cgtccaaggc 300

gaacccggcg gcggccgaca tggtggaccg catgctccgc ctgctcgcct cctacaacgt 360

cgtcaggtgc gagatggagg agggcgccga cggcaagctc tcccgccgct acgccgccgc 420

gccggtgtgc aagtggctga cgcccaacga ggacggcgtc tccatggccg ccctcgccct 480

catgaaccag gacaaggtcc tcatggagag ctggtactac cttaaggacg cagtcctgga 540

cggcggcatc ccgttcaaca aggcgtacgg gatgacggcg ttcgagtacc acggcacgga 600

cgcccgcttc aaccgcgtct tcaacgaggg catgaagaac cactccgtca tcatcaccaa 660

gaagctgctc gacctctaca ccggcttcga cgccgcctcc accgtcgtcg acgtcggcgg 720

cggcgtgggc gccactgtgg ccgccgtcgt ctcccgccac ccgcacatcc gggggatcaa 780

ctacgacctc ccccacgtca tctccgaggc gccgccgttc cccggggtgg agcacgtcgg 840

cggcgacatg ttcgcctccg tgccccgcgg cggcgacgcc atcctgatga agtggatcct 900

ccacgactgg agcgacgagc actgcgcgcg gctgctcaag aactgctacg acgcgctgcc 960

ggagcacggg aaggtggtgg tggtggagtg cgtgctgccg gagagctccg acgcgacggc 1020

gagggagcag ggggtgttcc acgtcgacat gatcatgctc gcccacaacc ccggcggcaa 1080
ggagaggtac gagagggagt tcagggagct cgcccgcgcc gccggattca ccggcttcaa 1140
ggccacctac atctacgcca acgcctgggc catcgagttc accaagtagg tgattggtga 1200
tcgatcgcca ttgttgagct cgatcaaggt gttcgaccat cgtcttcttc ttctcgtact 1260
tcttgttctt catccaaaag cgtgggtaat catgtcgttt gctgctggct gatgctgctg 1320
ctgcttgtgt tgtacttttg atgttcaatt ccggtgattc tgagttctaa tggatgtaac 1380
ctgtctgcta ttatatatat ataatcgaat atatgatata tttactcaaa aaaaaaaaaa 1440
aaaccatggt acccggatcc tcgaatt 1467

## Claims

1. (a) DNA comprising the nucleotide sequence of SEQ ID NO:6; or
(b) DNA having one or more nucleotides deleted, substituted or added in the nucleotide sequence of SEQ ID NO:6, the DNA being capable of expressing a protein having a naringenin-7-O-methyltransferase activity upon introduction into a rice cell.

2. A recombinant vector comprising the DNA of claim 1.

3. A host cell transformed with the recombinant vector of claim 2.

4. A plant transformant obtained by differentiation of a plant cell introduced with the recombinant vector of claim 2.

5. (a) DNA comprising the nucleotide sequence of SEQ ID NO:12; or
(b) DNA having one or more nucleotides deleted, substituted or added in the nucleotide sequence of SEQ ID NO:12, the DNA being capable of expressing a protein having a naringenin-7-O-methyltransferase activity upon introduction into a rice cell.

6. A recombinant vector comprising the DNA of claim 5.

7. A host cell transformed with the recombiant vector of claim 6.

8. A plant transformant obtained by differentiation of a plant cell introduced with the recombinant vector of claim 6.

9. (a) DNA comprising the nucleotide sequence of SEQ ID NO:2; or
(b) DNA having one or more nucleotides deleted, substituted or added in the nucleotide sequence of SEQ ID NO:2, the DNA having a promoter activity.

10. A recombinant vector comprising the DNA of claim 9.

11. A host cell transformed with the recombinant vector of claim 10.

12. (a) A protein comprising the amino acid sequence of SEQ ID NO:3; or
(b) a protein having one or more amino acids deleted, substituted or added in the nucleotide sequence of SEQ ID NO:3, and having a naringenin-7-O-methyltransferase activity.
